(19) **Europäisches Patentamt European Patent Office Office européen des brevets**

(11) **EP 1 625 131 B1**

(12) **EUROPEAN PATENT SPECIFICATION**

(45) Date of publication and mention of the grant of the patent:
**18.03.2009 Bulletin 2009/12**

(21) Application number: **04733202.8**

(22) Date of filing: **14.05.2004**

(51) Int Cl.:
*C07D 487/22* (2006.01)   *B01J 19/10* (2006.01)

(86) International application number:
**PCT/KR2004/001144**

(87) International publication number:
**WO 2004/101574 (25.11.2004 Gazette 2004/48)**

(54) **PROCESS AND APPARATUS FOR PREPARING METAL OR NONMETAL PHTHALOCYANINE**

VERFAHREN UND APPARAT ZUR HERSTELLUNG VON METALL- BZW. NICHTMETALLPHTHALOCYANIN

PROCEDE ET APPAREIL DE PREPARATION D'UN METAL OU DE PHTALOCYANINE NON METALLIQUE

(84) Designated Contracting States:
**AT BE BG CH CY CZ DE DK EE ES FI FR GB GR HU IE IT LI LU MC NL PL PT RO SE SI SK TR**

(30) Priority: **14.05.2003 KR 2003030727**
**14.05.2003 KR 2003030726**

(43) Date of publication of application:
**15.02.2006 Bulletin 2006/07**

(73) Proprietor: **Daehan Specialty Chemicals Co., Ltd. Ulsan City 689-892 (KR)**

(72) Inventors:
- **KWON, Jong Ho,**
  **106/1404, Bongmyeong I-PARK**
  **Cheongju City,**
  **Chungcheongbuk-do 361-300 (KR)**
- **JUNG, Ki Suck,**
  **102, Daedong Villa 4th**
  **Busan 608-811 (KR)**
- **SON, Woo Ho,**
  **71, Myoungji Prime Vil**
  **Busan 608-811 (KR)**
- **PARK, Seong Soo,**
  **302, 129-Dong, LG Metrocity**
  **Busan 608-890 (KR)**

(74) Representative: **Jacques, Philippe et al**
**Solvay (Société Anonyme)**
**Intellectual Property Department**
**Rue de Ransbeek, 310**
**1120 Bruxelles (BE)**

(56) References cited:
GB-A- 2 214 922          JP-A- 2003 048 892
US-B2- 6 791 796

- DATABASE CAPLUS [Online] CHEMICAL ABSTRACTS SERVICE, COLUMBUS, OHIO, US; JUNG, HYEON SEOK ET AL: "Process for synthesis of metallic and nonmetallic phthalocyanine in the absence of solvent using microwave energy" XP002405979 retrieved from STN Database accession no. 2004:948110 & KR 2003 022 831 A (S. KOREA) 17 March 2003 (2003-03-17)
- DATABASE CAPLUS [Online] CHEMICAL ABSTRACTS SERVICE, COLUMBUS, OHIO, US; JUNG, GI SEOK ET AL: "Process for preparing metallic and non-metallic phthalocyanine using microwave energy" XP002405980 retrieved from STN Database accession no. 2004:972810 & KR 2003 022 179 A (PHTHALOS CO., LTD., S. KOREA) 15 March 2003 (2003-03-15)
- DATABASE CAPLUS [Online] CHEMICAL ABSTRACTS SERVICE, COLUMBUS, OHIO, US; HU, ANDREW TEH ET AL: "Preparation of phthalocyanines by microwave irradiation" XP002405981 retrieved from STN Database accession no. 2005:201297 & TW 583 186 B (NATIONAL TSING HUA UNIVERSITY, TAIWAN; AGI CORPORATION) 11 April 2004 (2004-04-11)
- JUNG KI SUCK ET AL.: 'Conventional versus microwave synthesis of phthakicyanine material' JOURNAL OF MATERIALS SCIENCE LETTERS vol. 20, no. 24, 2001, pages 2203 - 2205, XP008075079

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

<u>TECHNICAL FIELD</u>

The present invention relates to a process and an apparatus for preparing a metal or nonmetal phthalocyanine using both microwave and ultrasonic wave energy in the absence or presence of a solvent.

<u>BACKGROUND ART</u>

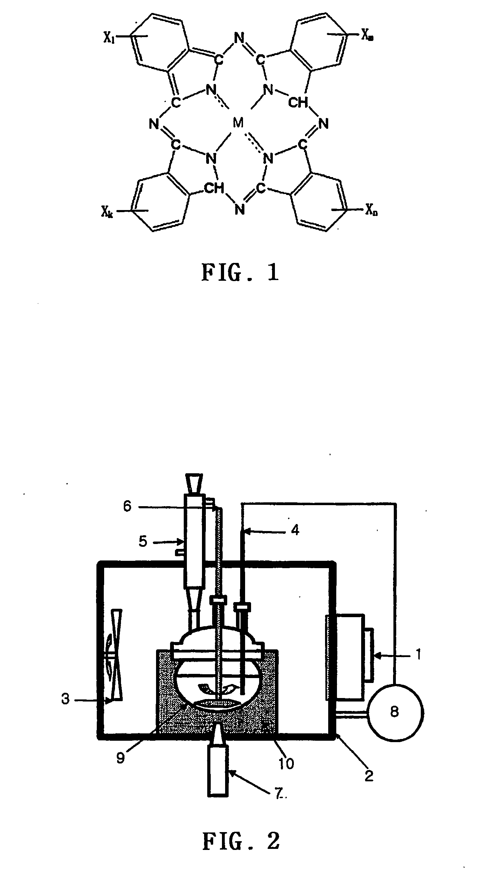

Phthalocyanines are compounds represented by the structural formula shown in Fig. 1, and exhibit superior stability and excellent photoelectric properties due to their unique chemical structure. For these reasons, phthalocyanines are currently used in a wide variety of applications such as dyes, pigments, chemical sensors, electrochromic displays, photovoltagic cells, radiators, photodisks, catalysts, nonlinear optics and the like.

A phthalocyanine is commonly prepared by reacting a starting material selected from anhydrous phthalic acid, phthalimide, 1,3-diiminoisoindoline, 1,2-dicyanobenzene and derivatives thereof with a metal chloride or an alkoxy metal using urea or ammonia as a nitrogen source at a temperature of 180˚C or higher, in the presence of a catalyst in an inert solvent or without any solvent.

The phthalocyanine thus prepared must essentially undergo pigmentation in order to be used as a pigment. The phthalocyanine pigmentation is mainly achieved by the following techniques:

1) Kneading: A phthalocyanine and finely divided salt or a metal salt are placed in a kneader, and are then kneaded for a predetermined period of time;
2) Milling and Organic solvent treatment: A phthalocyanine is subjected to dry or wet milling, and is then treated with an organic solvent; and
3) Milling and Kneading: A phthalocyanine is subjected to dry or wet milling, and is then kneaded.

Japanese Patent Laid-open No. Hei 8-291261 discloses a process for preparing a phthalocyanine using a heat source at 200~250˚C in the presence of a solvent such as chloronaphthalene. However, this process has the following problems: i) impurities that are difficult to remove are formed on a high-temperature portion due to the difference between internal and external temperatures of reactants, ii) since the phthalocyanine particles are non-uniformly dispersed and agglomerated in a needle shape, they must undergo long-term pigmentation before use as a pigment, iii) this process requires a large quantity of energy in order to recover the solvent used for the reaction, and iv) this process is disadvantageous in terms of process efficiency and environmental management.

Commonly, a phthalocyanine may be prepared using a conventional heat source in the absence of a solvent. This preparation process also has various problems. First, since reactants are not homogeneously mixed during preparation and are heated using electricity or thermal oil, the internal temperature of the reactor is non-uniform, causing low yield and poor quality of the phthalocyanine due to the presence of difficult-to-remove impurities formed at a high-temperature portion to which a relatively high heat is provided from the heat source. For these reasons, a number of solvent-free processes have been reported in the literatures. However, few processes have been applicable to mass production of phthalocyanines. Although some Czech and Chinese companies have attempted and finally succeeded in mass production of phthalocyanines, they stopped in the middle of production due to poor quality of the phthalocyanine undergoing pigmentation.

In order to solve the above-mentioned problems associated with non-uniform heat transfer, electricity and thermal oil as heat sources have been replaced with microwaves. Such trials can be found in many references (U.S. Patent No. 6,491,796; and Fifth International Electronic Conference on Synthetic Organic Chemistry (ECSOC-5), 1-30 September 2001, pp 4-5). Microwaves are electromagnetic waves having a wavelength ranging from 0.001m to 1m, and have functions such as rapid heating, selective heating and volume heating, etc. Since microwaves directly heat an object that is intended to be heated, external heating is unnecessary. Accordingly, the use of microwaves minimizes the formation of difficult-to-remove impurities. However, since reactants are not homogenously mixed during reaction despite the use of microwaves, the yield of phthalocyanines is not greatly increased, the mass production of phthalocyanines is difficult, and the quality of phthalocyanines is not comparable to that of phthalocyanines prepared by solvent processes. In conclusion, the preparation of a phthalocyanine using microwaves is not suitable for mass production and commercialization.

<u>SUMMARY OF THE INVENTION</u>

Therefore, the present invention has been made in view of the above problems of conventional solvent or solvent-free processes, e.g., long-term pigmentation, of conventional solvent processes using a heat source and microwave energy, and the present invention provides a process for preparing a metal or nonmetal phthalocyanine wherein both microwave and ultrasonic energy are used to enhance the yield, purity and physical properties of the phthalocyanine.

To this end, the present invention also provides an apparatus for preparing a metal or nonmetal phthalocyanine comprising: a magnetron providing a frequency of 0.1~100GHz and a power of 100~3,000W; a mode stirrer for making the wavelength of microwaves uniform in a microwave vessel; a PID temperature controller for accurately measuring and controlling the temperature of reactants; a microwave-shielded K-type thermocouple, a condenser and an agitator which are fitted into three holes formed on top of the microwave vessel, respectively; an ultrasonic tip fitted into a hole formed at bottom of the microwave vessel; a Pyrex container for accommodating reactants; and a solvent tank.

## DESCRIPTION OF DRAWINGS

The above and other objects, features and other advantages of the present invention will be more clearly understood from the following detailed description taken in conjunction with the accompanying drawings, in which:

Fig. 1 shows the structural formula of a metal or nonmetal phthalocyanine, or a derivative thereof (wherein M is copper, iron, cobalt, nickel, manganese, aluminum, gallium, vanadium, palladium, lead, tin, titanium, rubidium, terbium, cerium, lanthanum, zinc or hydrogen; X is hydrogen, fluoro, chloro, bromo, an alkyl group or alkoxy group; and k, 1, m and n are integers of 1 to 4);

Fig. 2 shows an apparatus for preparing a metal or nonmetal phthalocyanine using both microwave and ultrasonic wave energy, in accordance with the present invention;

Fig. 3 shows an electron micrograph (1,500x) of a copper phthalocyanine prepared by using a conventional solvent process;

Fig. 4 shows an electron micrograph (1,500x) of a copper phthalocyanine prepared by using a conventional solvent-free process; and

Fig. 5 shows an electron micrograph (1,500x) of a copper phthalocyanine prepared by using a conventional solvent-free process using microwave.

## DESCRIPTION OF PREFERRED EMBODIMENTS

The present invention will now be described in more detail.

The present invention provides a process for preparing a metal or nonmetal phthalocyanine by using both microwave and ultrasonic wave energy in the presence of a solvent.

Anhydrous phthalic acid, phthalimide, 1,3-diiminoisoindoline, 1,2-dicyanobenzene, a halogen derivative thereof, an alkyl derivative thereof, an alkoxy derivative thereof or the like is used as a starting material, and urea or ammonia is used as a nitrogen source. As a metal source suitable for use in the preparation of a metal phthalocyanine, a metal chloride (e.g., copper chloride, iron chloride, titanium chloride, etc.) or an alkoxy metal (e.g., ethoxy titanium, propoxy titanium, butoxy titanium, etc.) is used. As a reaction catalyst, ammonium molybdate, 1,8-diazabicyclo[5.4.0]undec-7-ene (DBU) or 1,5-diazabicyclo[4,3,0]-non-5-ene (DBN) is used. As a solvent, a halogenated aromatic hydrocarbon selected from alkyl benzenes, N-methyl-2-pyrrolidone, quinolines, trichlorobenzene and 1-chloronaphthalene, or an alcohol selected from isoamylalcohol, n-octanol, 2-ethylhexanol and ethyleneglycol, is used.

Fig. 2 shows an apparatus according to a first embodiment of the present invention. The apparatus comprises a magnetron 1 providing a frequency of 0.1~100GHz and a power of 100~3,000W; a mode stirrer 3 for making the wavelength of microwaves uniform in a microwave vessel 2; a PID temperature controller 8 made of stainless steel for accurately measuring. and controlling the temperature of reactants; a microwave-shielded K-type thermocouple 4, a condenser 5 and an agitator 6 which are fitted into three holes (diameter: about 1cm) formed on top of the microwave vessel, respectively; an ultrasonic tip 7 fitted into a hole (diameter: about 1cm) formed at the bottom of the microwave vessel; a Pyrex container 9 for accommodating reactants; and a solvent tank 10 filled with decalin (decahydronaphthalene) capable of transferring ultrasonic wave energy to the reactants without any reaction with microwaves.

In the apparatus of the present invention, the reactants are heated at a rate of about 2~20˚C/minute to 120˚C using microwave energy at a frequency of 0.1~100GHz and a power of 100~3,000W while being uniformly stirred in the presence of solvent, and are further heated at a rate of about 0.25~10˚C/minute to 130~250˚C, which is the final preparing temperature. During reaction, the temperature of the reactants can be accurately controlled using the PID temperature controller 8 within a deviation of ± 1˚C, and the microwave at a frequency of 0.1~100GHz and a power of 100~3,000W and ultrasonic wave energy at a frequency of 1~1,000kHz and a power of 100~5,000W are simultaneously used starting from the initial stage of the reaction.

According to the present invention, since the combination of microwave and ultrasonic wave energy can prevent agglomeration of phthalocyanine particles inside the reaction slurry and promote homogeneity of the slurry, metal or nonmetal phthalocyanine particles in a small needle shape can be prepared without agglomeration under the same preparing conditions of temperature and time. Accordingly, since the process of the present invention can considerably shorten the pigmentation time and enhance the quality of the phthalocyanine pigment, it is suitable for industrial applications.

In addition, the phthalocyanine produced by the process of the present invention can markedly shorten the time required for pigmentation. A phthalocyanine pigment obtained after long-term pigmentation, such as kneading or dry or wet milling, of a phthalocyanine prepared by a conventional solvent-free process is inferior in quality to a phthalocyanine pigment prepared by a conventional solvent-free process, and hence it cannot be practically used. As already reported in many references, since dry or wet milling can finely divide large particles and loosen firmly agglomerated particles, kneading time is shortened. In particular, such dry or wet milling is an essential step in the treatment of an organic solvent. Accordingly, already known processes further involve milling after preparation of phthalocyanines. In contrast, since the phthalocyanine prepared by the process of the present invention is milled immediately after preparation of the phthalocyanine, the phthalocyanine has a particle size by 50-60% smaller than that of phthalocyanine prepared by conventional processes. In addition, since the shape of the phthalocyanine particles prepared by the process of the present invention is almost spherical, further milling is unnecessary, the time required for pigmentation can be shortened by about 50% or more, and the phthalocyanine can be directly used as a pigment without undergoing additional pigmentation according to its intended application. Accordingly, a solvent-free process for preparing phthalocyanines, which has been thought to be impossible, can be put to practical use.

Pigmentation of the phthalocyanine prepared by the process of the present invention is carried out by the following techniques.

- Pigmentation 1: Kneading

A metal or nonmetal phthalocyanine and finely divided salt are charged into a kneader equipped with a sigma blade, and then an appropriate amount of diethylene glycol (DEG) is added thereto. The resulting mixture is kneaded at 100~110˚C for a predetermined period of time. After the kneaded mixture is taken out, it is dispersed in a 5% sulfuric acid solution, washed with distilled water at 90˚C until the pH is neutral, redispersed in distilled water, filtered, washed with distilled water at 90˚C until the electrical conductivity of the filtrate reaches 250☐S/cm or less, and dried in a dryer at about 105˚C for 24 hours.

- Pigmentation 2: Milling and organic solvent treatment

A metal or nonmetal phthalocyanine is introduced into an attritor or vibration mill, and then steel rods or balls are introduced thereinto. The phthalocyanine is dry milled for a predetermined period of time. Separately, a rosin solution is prepared in accordance with the procedure described in Example 1 of PCT publication WO 99/54410 (Applicant: Ciba Specialty Chemicals Holding Inc.). That is, an aqueous potassium hydroxide solution and rosin are added to a certain amount of water. The resulting mixture is completely dissolved to prepare a rosin solution, after which water is added for dilution. The milled phthalocyanine is dispersed in IPS2 (Charles Tennant, UK) as a solvent, and then the rosin solution is added thereto. The resulting mixture is refluxed for 4 hours. Thereafter, water is added to the refluxed mixture, and distilled to collect the solvent. Hydrochloric acid is added to the solvent-free slurry to render the slurry acidic. The slurry is filtered, washed until the pH is neutral, and dried in a dryer.

- Pigmentation 3: Milling and kneading

A phthalocyanine is introduced into an attritor or vibration mill, and then steel rods or balls are introduced thereinto. The phthalocyanine is dry milled for a predetermined period of time. The milled phthalocyanine is treated in the same manner as in Pigmentation 1.

The present invention will now be described in more detail with reference to the following examples and comparative examples.

Example 1

Preparation of copper phthalocyanine

This example was done in a solvent-type apparatus according to the present invention. Specifically, 42g of anhydrous phthalic acid, 49g of urea, 7g of copper chloride, 0.1g of ammonium molybdate and 100g of an alkylbenzene were introduced into a Pyrex container 9, and then the reactants were uniformly stirred at 180~185˚C for 3 hours by using microwaves at 28kHz and ultrasonic wave energy at 250W, to prepare a copper phthalocyanine. During reaction, the temperature of the reactants was accurately controlled using the PID temperature controller 8 within a deviation of ± 1˚C, and thus the microwave power was maintained at 100~3,000W. The microwave and ultrasonic wave energy were simultaneously used starting from the initial stage of the reaction. After completion of the preparation, the removal of the solvent was carried out by distillation at reduced pressure. The dried copper phthalocyanine was added to 500ml of a 5% sulfuric acid solution, acid-treated at 85˚C for one hour, washed with distilled water at 90˚C until the pH was neutral, alkali-treated with 500ml of a 1% aqueous sodium hydroxide solution at 85˚C for one hour, washed with distilled water at 90˚C until the pH was neutral, and dried in a dryer at about 105˚C for 24 hours.

Example 2

Preparation of other phthalocyanines

Various phthalocyanines were prepared in the same manner as in Example 1, except that 1,2-dicyanobenzene was used instead of anhydrous phthalic acid, and a metal salt as a metal source selected from titanium, iron, cobalt, aluminum, manganese, tin and nickel was used in the same equivalent weight instead of copper chloride (in the case of nonmetal phthalocyanine, the metal source was not used).

Example 3

Preparation of copper phthalocyanine

A copper phthalocyanine was prepared in the same manner as in Example 1, except that 41.2g of 1,3-diiminoisoindoline and 10g of urea were used instead of anhydrous phthalic acid and urea.

Example 4

Preparation of copper phthalocyanine

A copper phthalocyanine was prepared in the same manner as in Example 1, except that 36.3g of 1,2-dicyanobenzene and 10g of urea were used instead of anhydrous phthalic acid and urea.

Comparative Example 1

Preparation of copper phthalocyanine (conventional solvent process)
42g of anhydrous phthalic acid, 49g of urea, 7g of cuprous chloride, 0.1g of ammonium molybdate and 100g of an alkylbenzene were charged into a 1L three-neck glass flask equipped with a condenser, a thermometer and an agitator. The reactants were uniformly stirred at 180~185˚C for 3 hours to prepare a copper phthalocyanine. After completion of the preparation, the removal of the solvent was carried out by distillation at reduced pressure. The dried copper phthalocyanine was added to 500ml of a 5% sulfuric acid solution, acid-treated at 85˚C for one hour, washed with distilled water at 90˚C until the pH was neutral, alkali-treated with 500ml of a 1% aqueous sodium hydroxide solution at 85˚C for one hour, washed with distilled water at 90˚C until the pH was neutral, and dried in a dryer at about 105˚C for 24 hours.

Comparative Example 2

Preparation of copper phthalocyanine (Conventional solvent process)
A copper phthalocyanine was prepared in the same manner as in Example 1, except that microwave energy was not used.

Comparative Example 3

Preparation of copper phthalocyanine (Conventional solvent-free process)
A copper phthalocyanine was prepared in the same manner as in Comparative Example 1, except that alkylbenzene was not used as a solvent.

Comparative Example 4

Preparation of copper phthalocyanine (Conventional solvent-free process using microwave)
A copper phthalocyanine was prepared in the same manner as in Comparative Example 2, except that alkylbenzene was not used as a solvent.

The reaction yields of the copper phthalocyanines in Example 1 and Comparative Examples 1-4 are shown in Table 1 below.

Table 1

| Temperature (˚C) | Comparative Example 1 | Comparative Example 2 | Comparative Example 3 | Comparative Example 4 | Example 1 |
|---|---|---|---|---|---|
| | Yield (%) | Yield (%) | Yield (%) | Yield (%) | Yield (%) |
| 185 | 91 | 92 | 75 | 82 | 94 |

As can be seen from Table 1, the yield of the copper phthalocyanines prepared by the process of the present invention is higher than that of the copper phthalocyanines prepared by the conventional processes and the microwave processes in the absence or presence of a solvent.

The particle diameter and the particle size distribution of the copper phthalocyanines prepared in Example 1 and Comparative Examples 1 to 4 are shown in Table 2 below.

Table 2

| | Comparative Example 1 | Comparative Example 2 | Comparative Example 3 | Comparative Example 4 | Example 1 |
|---|---|---|---|---|---|
| Mv ($\mu$m) | 5.3 | 3.5 | 11.7 | 7.5 | 2.8 |
| $d_{10}$ ($\mu$m) | 0.9 | 0.8 | 1.3 | 1.0 | 0.7 |
| $d_{50}$ ($\mu$m) | 3.2 | 2.9 | 6.5 | 5.2 | 1.8 |
| $d_{90}$ ($\mu$m) | 13.5 | 10.8 | 23.6 | 15.8 | 7.9 |
| Note) mv = mean value | | | | | |

As evident from Table 2, the copper phthalocyanine prepared by the process of the present invention has a uniform particle diameter and a narrow particle size distribution, compared to the copper phthalocyanines prepared by the conventional processes and the microwave processes in the absence or presence of a solvent.

The results of Table 3 below clearly demonstrate that the yields of the metal and nonmetal phthalocyanines prepared in Examples 1 and 2 are relatively high.

Table 3

| Metal | Cu | Ti | Fe | Co | Al | Mn | Sn | Ni | H |
|---|---|---|---|---|---|---|---|---|---|
| Yield | ◎ | ◎ | ◎ | ◎ | ◎ | ◎ | ◎ | ◎ | 0 |
| Note) ◎: Very high yield, ○: relatively high yield | | | | | | | | | |

Comparative Example 5

Preparation of copper phthalocyanine (conventional solvent-free process)

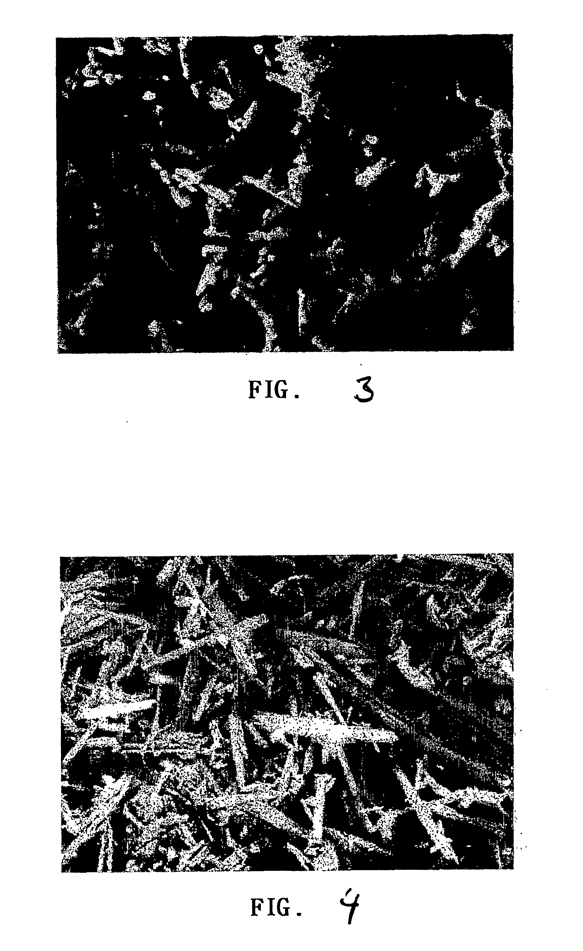

42g of anhydrous phthalic acid, 49g of urea, 7g of cuprous chloride and 0.1g of ammonium molybdate were charged into a 1L three-neck glass flask equipped with a condenser, a thermometer and an agitator. The reactants were heated at a rate of 10˚C/minute to 120˚C with agitating at 300~400rpm, and were further heated at a rate of 5˚C/minute to a final preparing temperature (180˚C). While the final preparing temperature was maintained for 3 hours, the reactants were uniformly agitated to prepare a copper phthalocyanine. After completion of the preparation, 500ml of a 5% sulfuric acid solution was introduced into the flask. After the resulting mixture was agitated for 30 minutes, it was taken out. The resulting copper phthalocyanine slurry was subjected to acid-treatment at 85˚C for one hour, filtered, and washed with distilled water at 90˚C until the pH was neutral. The acid-treated copper phthalocyanine was redispersed in 500ml of a 1% aqueous sodium hydroxide solution and was then alkali-treated at 85˚C for one hour. The alkali-treated copper phthalocyanine was filtered, washed with distilled water at 90˚C until the pH was neutral, and dried in a dryer at about 105˚C for 24 hours. Fig. 4 shows an electron micrograph (1,500x) of the copper phthalocyanine prepared by the conventional solvent-free process.

Comparative Example 6

Preparation of copper phthalocyanine (solvent-free process using microwave)

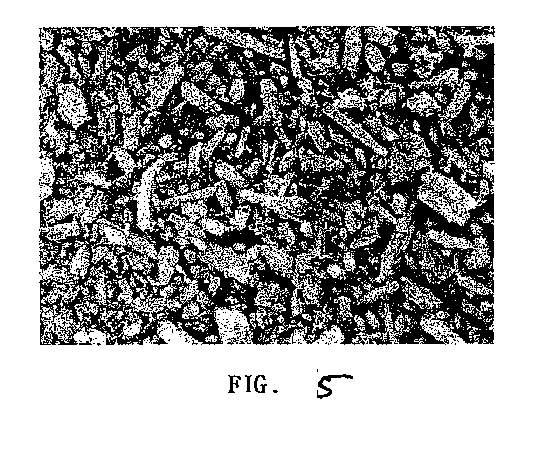

A copper phthalocyanine was prepared in the same manner as in Comparative Example 1, except that the microwave generation apparatus (2.45GHz, 100~3,000W) shown in Fig. 2 was used instead of the three-neck glass flask. Fig. 5 shows an electron micrograph (1,500x) of the copper phthalocyanine prepared by the conventional solvent-free process using microwave.

Comparative Example 7

Preparation of copper phthalocyanine (solvent process)

A copper phthalocyanine was prepared in the same manner as in Comparative Example 1, except that 100ml of AS-P2 (Nippon Petrochemical, Japan) was used as a solvent. Fig. 3 shows an electron micrograph (1,500x) of the copper phthalocyanine prepared by the conventional solvent process.

The purity and reaction yield of the copper phthalocyanine were measured as follows.

<Purity>

"A"g of a copper phthalocyanine is dissolved in concentrated sulfuric acid, and then the resulting sulfuric acid solution is diluted in distilled water to recrystallize the copper phthalocyanine. The copper phthalocyanine crystal is filtered through a glass filter (2G4) weighing "B"g, washed with distilled water until the pH is neutral, redispersed in a 3% aqueous ammonia, filtered, washed with distilled water until the pH is neutral, and dried in a dryer at about 105°C for 24 hours. Thereafter, the glass filter is placed in a desiccator to allow it to cool to room temperature, and weighed ("C"g).

The purity of the copper phthalocyanine is calculated according to the following equation:

$$Purity(\%) = \frac{(C\text{-}B)}{A} * 100$$

<Reaction Yield>

First, the weight (A) of a crude copper phthalocyanine prepared through preparing and purification is multiplied by the purity (B). The obtained product is divided by the molecular weight (C) of the copper phthalocyanine to obtain a mole number (D) of the copper phthalocyanine, the mole number (D) is divided by a value obtained by dividing the mole number (E) of anhydrous phthalic acid (or its derivative) added as a starting material by 4, and then the resulting value is multiplied by 100 to determine the reaction yield of the copper phthalocyanine.

$$Mole number(D) \ of \ copper \ phthalocyanine = \frac{A * (\frac{B}{100})}{C}$$

$$Yield(\%) = \frac{D}{(\frac{E}{4})} * 100$$

The purity and reaction yield of the copper phthalocyanines prepared in Example 1 and Comparative Examples 1 to 3 are shown in Table 4 below.

Table 4

| Temperature (°C) | Comparative Example 1 | | Comparative Example 2 | | Example 1 | | Comparative Example 3 | |
|---|---|---|---|---|---|---|---|---|
| | Yield | Purity | Yield | Purity | Yield | Purity | Yield | Purity |
| | (%) | (%) | (%) | (%) | (%) | (%) | (%) | (%) |
| 180 - 185 | 75 | 93 | 82 | 94 | 90 | 97 | 91 | 97 |

As can be seen from Table 4, the solvent-free process using the milling-type microwave apparatus enables the preparation of copper phthalocyanines with a high purity in high yield comparable to the conventional solvent processes.

Example 5

Preparation of copper phthalocyanine pigment (kneading)

50g of each of the copper phthalocyanines prepared in Example 1 and Comparative Examples 1 to 7, 300g of finely divided salt and 50g of diethylene glycol (DEG) were charged into a kneader, and then the resulting mixture was kneaded at 100~110°C for 4, 6 and 8 hours, respectively, to prepare copper phthalocyanine pigments. After kneading, each of the kneaded mixtures was taken out, dispersed in a 5% sulfuric acid solution, filtered, washed with distilled water at 90°C until the pH was neutral, redispersed in distilled water, filtered, washed with distilled water at 90°C until the electrical conductivity of the filtrate reached 250□s/cm or less, and dried in a dryer at about 105°C for 24 hours.

Example 6

Preparation of copper phthalocyanine pigment (kneading and organic solvent treatment)

100g of each of the copper phthalocyanines prepared in Examples 1 to 4 and Comparative Examples 1 to 7 was charged into a vibration mill (CHUOKAKOKI, Japan) filled with 14kg of steel rods having a diameter of 15mm, and was then milled for 60, 90 and 120 minutes, respectively. Separately, 15.3g of a 50% aqueous potassium hydroxide and 40g of rosin were added to 250g of water, and completely dissolved to prepare a rosin solution. Water was added to the rosin solution until the total volume reached 267mL. 70g of the milled copper phthalocyanine was dispersed in 200mL of IPS2 (CHARLES TENNANT, UK) as a solvent, and then 10.5g of the rosin solution was added thereto. The mixture was refluxed for 4 hours. Thereafter, 200ml of water was added to the refluxed mixture, and distilled to collect the solvent. 30ml of a 36% hydrochloric acid solution was added to the solvent-free slurry to render the slurry acidic. The slurry was filtered, washed until the pH was neutral, and dried in a dryer at 75°C.

Example 7

Preparation of copper phthalocyanine pigment (Milling + kneading)

100g of each of the copper phthalocyanines prepared in Examples 1 to 4 and Comparative Examples 1 to 7 was charged into a vibration mill (CHUOKAKOKI, Japan) filled with 14kg of steel rods having a diameter of 15mm, and was then milled for 60 minutes. 50g of the milled copper phthalocyanines, 300g of finely divided salt and 50g of diethylene glycol (DEG) were charged into a kneader, and then the resulting mixture was kneaded at 100~110°C for 4, 6 and 8 hours, respectively, to prepare copper phthalocyanine pigments.
The quality of the copper phthalocyanine pigments prepared by the pigmentation processes was evaluated by the following tests and graded based on the following criteria.

| Grade | Sharpness (dC) | Color density (%) |
|---|---|---|
| /=/ | 0.00~0.10 | 0~1 |
| 1 | 0.11~0.30 | 1~2 |
| 2 | 0.31~0.18 | 2~5 |
| 3 | 0.81~1.40 | 5~10 |
| 4 | 1.41~2.20 | 10~20 |
| 5 | 2.21~3.00 | 20~40 |
| 6 | 3.01~ | 40~ |

(continued)

| Grade | Sharpness (dC) | Color density (%) |
|---|---|---|
| + | Sharp | High |
| - | Not sharp | Low |

Test 1. Oil ink test

A copper phthalocyanine and a copper phthalocyanine pigment were mixed to have the composition indicated below:

Copper phthalocyanine (pigment): 10g
Oil ink resin (Rosin Modified Phenolic Resin): 40g
The mixture was dispersed twice using a three-roll mill, and then the color and the dispersability were evaluated.

0.3g of the dark ink sample thus obtained and 3g of a white ink were homogeneously mixed to prepare a colored ink sample, and then the color was evaluated.

Test 2. Dispersability test

The degree of dispersion of the copper phthalocyanines and the copper phthalocyanine pigments was tested and evaluated from the dark ink samples obtained in Test 1 using a Grind-O-Meter.

The oil ink test and the dispersablity test of the copper phthalocyanines in the examples and comparative examples, and the copper phthalocyanine pigments prepared by the pigmentation processes of the present invention were conducted, and the results are shown in Tables 5 to 8 below.

Table 5

| Preparative Example No. of copper phthalocyanine | Test results of color and physical properties | | | |
|---|---|---|---|---|
| | Dark sample | | Colored sample | |
| | Dispersability ($\mu$m) | Sharpness | Sharpness | Color density |
| Example 1 | 9 | 1+ | 2+ | 2+ |
| Example 3 | 15 | /=/ | /=/ | /=/ |
| Example 4 | 15 | /=/ | /=/ | /=/ |
| Comparative Example 1 | 13 | standard | standard | standard |
| Comparative Example 2 | 12 | /=/ | 1+ | 1+ |
| Comparative Example 3 | 75 | 6- | 6- | /=/ |
| Comparative Example 4 | 60 | 6- | 6- | /=/ |
| Comparative Example 5 | 75 | 6- | 6- | /=/ |
| Comparative Example 6 | 60 | 6- | 6- | /=/ |
| Comparative Example 7 | 13 | standard | standard | standard |

EP 1 625 131 B1

Table 6

| Preparative Example No. of copper phthalocyanine | Test results of color and physical properties | | | | | | | | | | | |
|---|---|---|---|---|---|---|---|---|---|---|---|---|
| | Kneading for 4 hours | | | | Kneading for 6 hours | | | | Kneading for 8 hours | | | |
| | Dark sample | | Colored sample | | Dark sample | | Colored sample | | Dark sample | | Colored sample | |
| | Dispersability (μm) | dC | dC | Color density | Dispersability (μm) | dC | dC | Color density | Dispersability (μm) | dC | dC | Color density |
| Example 1 | < 5 | 2+ | 2+ | 2+ | < 5 | 3+ | 3+ | 3+ | < 5 | 2+ | 2+ | 2+ |
| Example 3 | 7 | 2+ | 2+ | 2+ | <5 | 1+ | 1+ | /=/ | <5 | 1+ | 1+ | /=/ |
| Example 4 | 6 | 2+ | 2+ | 2+ | <5 | 1+ | 1+ | /=/ | <5 | 1+ | 1+ | /=/ |
| Comparative Example 1 | 7 | standard | | | < 5 | standard | | | < 5 | standard | | |
| Comparative Example 2 | < 5 | /=/ | /=/ | /=/ | < 5 | 1+ | 1+ | /=/ | < 5 | 1+ | 1+ | /=/ |
| Comparative Example 3 | 55 | 6- | 6- | 1- | 55 | 6- | 6- | 1- | 45 | 6- | 6- | 1- |
| Comparative Example 4 | 50 | 6- | 6- | 1- | 50 | 6- | 6- | 1- | 45 | 6- | 6- | 1- |
| Comparative Example 5 | 55 | 6- | 6- | 1- | 55 | 6- | 6- | 1- | 45 | 6- | 6- | 1- |
| Comparative Example 6 | 50 | 6- | 6- | 1- | 55 | 6- | 6- | 1- | 45 | 6- | 6- | 1- |
| Comparative Example 7 | 7 | standard | | | <5 | standard | | | <5 | standard | | |

Table 7

| Preparative Example No. of copper phthalocyanine | Test results of color and physical properties | | | | | | | | | | | |
|---|---|---|---|---|---|---|---|---|---|---|---|---|
| | Milling for 60 min. | | | | Milling for 90 min. | | | | Milling for 120 min. | | | |
| | Dark sample | | Colored sample | | Dark·sample | | Colored sample | | Dark sample | | Colored sample | |
| | Dispersability (μm) | dC | dC | Color density | Dispersability (μm) | dC | dC | Color density | Dispersability (μm) | dC | dC | Color density |
| Example 1 | < 5 | 1+ | 1+ | 1+ | < 5 | 3+ | 3+ | 3+ | < 5 | 2+ | 2+ | 2+ |
| Example 3 | 8 | 3+ | 3+ | 2+ | < 5 | 2+ 1+ | | 1+ | < 5 | 1+ | 1+ | 1+ |
| Example 4 | 7 | 3+ | 3+ | 2+ | < 5 | 2+ | 1+ | 1+ | < 5 | 1+ | 1+ | 1+ |
| Comparative Example 1 | 7 | standard | | | < 5 | standard | | | < 5 | standard | | |
| Comparative Example 2 | < 5 | /=/ | /=/ | /=/ | < 5 | 1+ | 1+ | /=/ | < 5 | 1+ | 1+ | /=/ |
| Comparative Example 3 | 50 | 6- | 6- | 1- | 50 | 6- | 6- | 1- | 45 | 6- | 6- | 1- |
| Comparative Example 4 | 40 | 6- | 6- | 1- | 35 | 6- | 6- | 1- | 35 | 6- | 6- | 1- |
| Comparative Example 5 | 50 | 6- | 6- | 1- | 50 | 6- | 6- | 1- | 45 | 6- | 6- | 1- |
| Comparative Example 6 | 40 | 6- | 6- | 1- | 35 | 6- | 6- | 1- | 35 | 6- | 6- | 1- |
| Comparative Example 7 | 7 | standard | | | <5 | standard | | | <5 | standard | | |

EP 1 625 131 B1

Table 8

| Preparative Example No. of copper phthalocyanine | Test results of color and physical properties | | | | | | | | | | | |
|---|---|---|---|---|---|---|---|---|---|---|---|---|
| | Kneading for 2 hours | | | | Kneading for 4 hours | | | | Kneading for 6 hours | | | |
| | Dark sample | | Colored sample | | Dark sample | | Colored sample | | Dark sample | | Colored sample | |
| | Dispersability ($\mu$m) | dC | dC | Color density | Dispersability ($\mu$m) | dC | dC | Color density | Dispersability ($\mu$m) | dC | dC | Color density |
| Example 1 | <5 | 1+ | 1+ | 1+ | < 5 | 3+ | 3+ | 3+ | < 5 | 2+ | 2+ | 2+ |
| Example 3 | 9 | 2+ | 2+ | 1+ | < 5 | 1+ | 2+ | /=/ | < 5 | 1+ | 1+ | /=/ |
| Example 4 | 11 | 2+ | 2+ | 1+ | < 5 | 1+ | 2+ | /=/ | < 5 | 1+ | 1+ | /=/ |
| Comparative Example 1 | 8 | standard | | | < 5 | standard | | | < 5 | standard | | |
| Comparative Example 2 | < 5 | /=/ | /=/ | /=/ | < 5 | 1+ | 1+ | /=/ | < 5 | 1+ | 1+ | /=/ |
| Comparative Example 3 | 60 | 6- | 6- | 1- | 55 | 6- | 6- | 1- | 55 | 6- | 6- | 1- |
| Comparative Example 4 | 60 | 6- | 6- | 1- | 55 | 6- | 6- | 1- | 50 | 6- | 6- | 1- |
| Comparative Example 5 | 60 | 6- | 6- | 1- | 55 | 6- | 6- | 1- | 55 | 6- | 6- | 1- |
| Comparative Example 6 | 60 | 6- | 6- | 1- | 55 | 6- | 6- | 1- | 50 | 6- | 6- | 1- |
| Comparative Example 7 | 8 | standard | | | <5 | sta ndard | | | <5 | standard | | |

Test 3. Paint test

A copper phthalocyanine and a copper phthalocyanine pigment was mixed in accordance with the compositions indicated below:

Glass balls: 100g
Transparent paint resin (Alkyd/melamine resin): 50g
Copper phthalocyanine (pigment): 3g

The mixture was placed in a plastic tub, and dispersed in a paint mill for 45 minutes to prepare a dark paint sample. The color of the paint sample was evaluated.

5g of the dark paint sample was mixed with 20g of a white paint to prepare a colored paint sample. The color of the colored paint sample was evaluated. The dark and colored paint samples were applied onto a paint-extension paper using a paint extender, dried in a dryer, and then their colors were evaluated. The results are shown in Tables 9 to 11 below.

Table 9

| Preparative Example No. of copper phthalocyanine | Test results of color and physical properties | | | | | | | | |
|---|---|---|---|---|---|---|---|---|---|
| | Kneading for 4 hours | | | Kneading for 6 hours | | | Kneading for 8 hours | | |
| | Dark sample | Colored sample | | Dark sample | Colored sample | | Dark sample | Colored sample | |
| | dC | dC | Color density | dC | dC | Color density | dC | dC | Color density |
| Example 1 | 3+ | 3+ | 3+ | 4+ | 4+ | 4+ | 3+ | 3+ | 3+ |
| Example 3 | 1+ | 2+ | 2+ | 1+ | 1+ | /=/ | 1+ | 1+ | /=/ |
| Example 4 | 1+ | 2+ | 2+ | 1+ | 1+ | /=/ | 1+ | 1+ | /=/ |
| Comparative Example 1 | standard | | | standard | | | standard | | |
| Comparative Example 2 | /=/ | /=/ | /=/ | /=/ | /=/ | /=/ | 1+ | 1+ | /=/ |
| Comparative Example 3 | 6- | 6- | 1- | 6- | 6- | 1- | 6- | 6- | 1- |
| Comparative Example 4 | 6- | 6- | 1- | 6- | 6- | 1- | 6- | 6- | 1- |
| Comparative Example 5 | 6- | 6- | 1- | 6- | 6- | 1- | 6- | 6- | 1- |
| Comparative Example 6 | 6- | 6- | 1- | 6- | 6- | 1- | 6- | 6- | 1- |
| Comparative Example 7 | standard | | | standard | | | standard | | |

Table 10

| Preparative Example No. of copper phthalocyanine | Test results of color and physical properties | | | | | | | | |
|---|---|---|---|---|---|---|---|---|---|
| | Milling for 60 min. | | | Milling for 90 min. | | | Milling for 120 min. | | |
| | Dark sample | Colored sample | | Dark sample | Colored sample | | Dark sample | Colored sample | |
| | dC | dC | Color density | dC | dC | Color density | dC | dC | Color density |
| Example 1 | 3+ | 3+ | 3+ | 4+ | 4+ | 4+ | 3+ | 3+ | 3+ |
| Example 3 | 2+ | 3+ | 3+ | 1+ | 2+ | 2+ | 2+ | 2+ | 2+ |
| Example 4 | 2+ | 3+ | 3+ | 1+ | 2+ | 2+ | 2+ | 2+ | 2+ |
| Comparative Example 1 | standard | | | standard | | | standard | | |
| Comparative Example 2 | /=/ | /=/ | /=/ | /=/ | /=/ | /=/ | 1+ | 1+ | /=/ |
| Comparative Example 3 | 6- | 6- | 1- | 6- | 6- | 1- | 6- | 6- | 1- |
| Comparative Example 4 | 6- | 6- | 1- | 6- | 6- | 1- | 6- | 6- | 1- |
| Comparative Example 5 | 6- | 6- | 1- | 6- | 6- | 1- | 6- | 6- | 1- |
| Comparative Example 6 | 6- | 6- | 1- | 6- | 6- | 1- | 6- | 6- | 1- |
| Comparative Example 7 | standard | | | standard | | | standard | | |

Table 11

| Preparative Example No. of copper phthalocyanine | Test results of color and physical properties | | | | | | | | |
|---|---|---|---|---|---|---|---|---|---|
| | Kneading for 2 hours | | | Kneading for 4 hours | | | Kneading for 6 hours | | |
| | Dark sample | Colored sample | | Dark sample | Colored sample | | Dark sample | Colored sample | |
| | dC | dC | Color density | dC | dC | Color density | dC | dC | Color density |
| Example 1 | 2+ | 2+ | 2+ | 3+ | 3+ | 3+ | 2+ | 2+ | 2+ |
| Example 3 | 2+ | 3+ | 2+ | 1+ | 2+ | 1+ | 2+ | 2+ | /=/ |
| Example 4 | 2+ | 3+ | 2+ | 1+ | 2+ | 1+ | 2+ | 2+ | /=/ |
| Comparative Example 1 | standard | | | standard | | | standard | | |
| Comparative Example 2 | /=/ | /=/ | /=/ | /=/ | /=/ | /=/ | 1+ | 1+ | /=/ |

(continued)

| Preparative Example No. of copper phthalocyanine | Test results of color and physical properties | | | | | | | | |
|---|---|---|---|---|---|---|---|---|---|
| | Kneading for 2 hours | | | Kneading for 4 hours | | | Kneading for 6 hours | | |
| | Dark sample | Colored sample | | Dark sample | Colored sample | | Dark sample | Colored sample | |
| | dC | dC | Color density | dC | dC | Color density | dC | dC | Color density |
| Comparative Example 3 | 6- | 6- | 1- | 6- | 6- | 1- | 6- | 6- | 1- |
| Comparative Example 4 | 6- | 6- | 1- | 6- | 6- | 1- | 6- | 6- | 1+ |
| Comparative Example 5 | 6- | 6- | 1- | 6- | 6- | 1- | 6- | 6- | 1+ |
| Comparative Example 6 | 6- | 6- | 1- | 6- | 6- | 1- | 6- | 6- | 1+ |
| Comparative Example 7 | standard | | | standard | | | standard | | |

As is apparent from the above tables, the copper phthalocyanine prepared by the conventional solvent-free process (Comparative Example 5) and the copper phthalocyanine prepared by the microwave solvent-free process (Comparative Example 6) are very poor in dispersability, sharpness (dC) and color density, compared to the copper phthalocyanine prepared by the solvent process (Comparative Example 7).

In addition, the pigment obtained by the pigmentation of the copper phthalocyanine prepared by the conventional solvent-free process (Comparative Example 5) and the pigment obtained by the pigmentation of the copper phthalocyanine prepared by the microwave solvent-free process (Comparative Example 6) are very poor in sharpness, color density and dispersability, compared to the pigment obtained by the pigmentation of the copper phthalocyanine prepared by the solvent process (Comparative Example 7) under the same conditions.

INDUNDUSTIRAL APPLICABILITY

As apparent from the above description, according to the present invention, since the combination of microwave and ultrasonic wave energy in the presence of a solvent can prevent agglomeration inside the reaction slurry and promote homogeneity of the slurry, uniform metal or nonmetal phthalocyanine particles in a small needle shape can be prepared without agglomeration under the same preparing conditions of temperature and time. Accordingly, the time required for pigmentation can be considerably shorten. Furthermore, since the apparatus of the present invention can solve a problem, i.e. poor quality of pigments of copper phthalocyanines prepared by solvent-free processes than pigments of copper phthalocyanines prepared by solvent processes despite long-term pigmentation, it enables preparation of pigments having a comparable quality to pigments of phthalocyanine prepared by solvent processes and a sharpness superior to the phthalocyanines prepared by solvent processes by short-term pigmentation.

**Claims**

1. A process for preparing a metal or nonmetal phthalocyanine by treating anhydrous phthalic acid, phthalimide, 1,3-diiminoisoindoline, 1,2-dicyanobenzene, an halogen derivative thereof, an alkyl derivative thereof or an alkoxy derivative thereof with microwaves and ultrasonic waves, optionally in the presence of a metal chloride or an alkoxy metal.

2. The process according to claim 1 in which a solvent is used.

3. The process according to claim 1 carried out in the absence of a solvent.

4. The process according to claim 1 in which the metal is selected from the group consisting of copper, iron, nickel, cobalt, manganese, aluminum, palladium, tin, lead, titanium, rubidium, vanadium, gallium, terbium, cerium, lanthanum and zinc.

5. The process according to claim 1 or 4 in which the metal is copper.

6. The process according to claim 1 or 4 in which urea is used as a nitrogen source.

7. The process according to claim 1 or 4 in which the reacting is carried out under urea or ammonia atmosphere.

8. The process according to claim 1 or 4 in which the reacting is carried out in the presence of a catalyst selected from ammonium molybdate, DBU and DBN.

9. The process according to claim 1 or 2 in which the solvent is a halogenated aromatic hydrocarbon selected from alkyl benzenes, N-methyl-2-pyrrolidone, quinolines, trichlorobenzene and 1-chloronaphthalene, or an alcohol selected from isoamylalcohol, n-octanol, 2-ethylhexanol and ethyleneglycol.

10. The process according to claim 1 or 4, comprising heating at a rate of about 2~20˚C/minute to 120˚C using microwave energy, and further heating at a rate of about 0.25~10˚C/minute to a final preparing temperature of 130~250˚C.

11. An apparatus for preparing a metal or nonmetal phthalocyanine comprising: a magnetron (1) providing a frequency of 0.1~100GHz and a power of 100~3,000W; a mode stirrer (3) for making the wavelength of microwaves uniform in a microwave vessel (2); a PID temperature controller (8) for accurately measuring and controlling the temperature of reactants; a microwave-shielded K-type thermocouple (4) a condenser (5) and an agitator (6) which are fitted into three holes formed on top of the microwave vessel (2), respectively; an ultrasonic tip (7) fitted into a hole formed at bottom of the microwave vessel (2); a Pyrex container (9) for accommodating reactants; and a solvent tank (10), in which anhydrous phthalic acid, phthalimide, 1,3-diiminoisoindoline, 1,2-dicyanobenzene, an halogen derivative thereof, an alkyl derivative thereof or an alkoxy derivative thereof is homogeneously mixed with a metal chloride or an alkoxy metal in a solvent in the Pyrex container (9) at 130~250˚C for 0.25~15 hours by using microwave at a frequency of 0.1~100GHz and a power of 100~3,000W and ultrasonic wave at a frequency of 1~1,000GHz and a power of 100~5,000W, while accurately controlling the temperature of the reactants using the K-type thermocouple (4) and the PID temperature controllers (8).

12. The apparatus according to claim 11 in which the metal is selected from the group consisting of copper, iron, nickel, cobalt, manganese, aluminum, palladium, tin, lead, titanium, rubidium, vanadium, gallium, terbium, cerium, lanthanum and zinc.

13. The apparatus according to claim 11 in which the metal is copper.

14. The apparatus according to claim 11 in which urea is used as a nitrogen source.

15. The apparatus according to claim 11 carried out under urea or ammonia atmosphere.

16. The apparatus according to claim 11 carried out in the presence of a catalyst selected from ammonium molybdate, DBU and DBN.

17. The apparatus according to claim 11 in which the solvent is a halogenated aromatic hydrocarbon selected from alkyl benzenes, N-methyl-2-pyrrolidone, quinolines, trichlorobenzene and 1-chloronaphthalene, or an alcohol selected from isoamylalcohol, n-octanol, 2-ethylhexanol and ethyleneglycol.

18. The apparatus according to claim 11 in which the mixture is heated at a rate of about 2~20˚C/minute to 120˚C using microwave energy, and is further heated at a rate of about 0.25~10˚C/minute to a final preparing temperature of 130~250˚C.

**Patentansprüche**

1. Verfahren zur Herstellung eines Metall- oder Nichtmetallphthalocyanins, bei dem man wasserfreie Phthalsäure, Phthalimid, 1,3-Diiminoisoindolin, 1,2-Dicyanobenzol, ein halogeniertes Derivat davon, ein Alkylderivat davon oder ein Alkoxyderivat davon mit Mikrowellen und Ultraschallwellen behandelt, gegebenenfalls in Gegenwart eines Metallchlorids oder eines Alkoxymetalls.

2. Verfahren nach Anspruch 1, bei dem man ein Lösungsmittel verwendet.

3. Verfahren nach Anspruch 1, das ohne Lösungsmittel durchgeführt wird.

4. Verfahren nach Anspruch 1, bei dem man das Metall aus der Gruppe bestehend aus Kupfer, Eisen, Nickel, Cobalt, Mangan, Aluminium, Palladium, Zinn, Blei, Titan, Rubidium, Vanadium, Gallium, Terbium, Cer, Lanthan und Zink auswählt.

5. Verfahren nach Anspruch 1 oder 4, bei dem es sich bei dem Metall um Kupfer handelt.

6. Verfahren nach Anspruch 1 oder 4, bei dem man Harnstoff als Stickstoffquelle verwendet.

7. Verfahren nach Anspruch 1 oder 4, bei dem man die Umsetzung unter Harnstoff- oder Ammoniakatmosphäre durchführt.

8. Verfahren nach Anspruch 1 oder 4, bei dem man die Umsetzung in Gegenwart eines unter Ammoniummolybdat, DBU und DBN ausgewählten Katalysators durchführt.

9. Verfahren nach Anspruch 1 oder 2, bei dem es sich bei dem Lösungsmittel um einen halogenierten aromatischen Kohlenwasserstoff, ausgewählt unter Alkylbenzolen, N-Methyl-2-pyrrolidon, Chinolinen, Trichlorbenzol und 1-Chlornaphthalin, oder einen Alkohol, ausgewählt unter Isoamylalkohol, n-Octanol, 2-Ethylhexanol und Ethylenglykol, handelt.

10. Verfahren nach Anspruch 1 oder 4, bei dem man unter Verwendung von Mikrowellenenergie mit einer Rate von etwa 2~20˚C/Minute auf 120˚C erhitzt und ferner mit einer Rate von etwa 0,25~10˚C/Minute auf eine Endherstellungstemperatur von 130~250˚C erhitzt.

11. Vorrichtung zur Herstellung eines Metall- oder Nichtmetallphthalocyanins mit: einem Magnetron (1), das eine Frequenz von 0,1~100 GHz und eine Leistung von 100~3.000 W bereitstellt; einem Modenrührer (3) zum Einheitlichmachen der Wellenlänge von Mikrowellen in einem Mikrowellenbehälter (2); einem PID-Temperaturregler (8) zur genauen Messung und Regelung der Temperatur von Reaktanten; einem gegenüber Mikrowellen abgeschirmten K-Typ-Thermoelement (4), einem Kondensator (5) und einem Rührer (6), die in drei an der Oberseite des Mikrowellenbehälters (2) ausgebildete Löcher eingepaßt sind; einer Ultraschallspitze (7), die in ein an der Unterseite des Mikrowellenbehälters (2) ausgebildetes Loch eingepaßt ist; einem Pyrex-Behälter (9) zur Aufnahme von Reaktanten und einem Lösungsmitteltank (10), in dem wasserfreie Phthalsäure, Phthalimid, 1,3-Diiminoisoindolin, 1,2-Dicyanobenzol, ein halogeniertes Derivat davon, ein Alkylderivat davon oder ein Alkoxyderivat davon in dem Pyrex-Behälter (9) durch Verwendung von Mikrowellen bei einer Frequenz von 0,1~100 GHz und einer Leistung von 100~3.000 W und Ultraschallwellen mit einer Frequenz von 1~1.000 GHz und einer Leistung von 100~5.000 W über einen Zeitraum von 0,25~15 Stunden bei 130~250˚C in einem Lösungsmittel mit einem Metallchlorid oder einem Alkoxymetall homogen gemischt wird, wobei die Temperatur der Reaktanten unter Verwendung des K-Typ-Thermoelements (4) und des PID-Temperaturreglers (8) genau geregelt wird.

12. Vorrichtung nach Anspruch 11, in der das Metall aus der Gruppe bestehend aus Kupfer, Eisen, Nickel, Cobalt, Mangan, Aluminium, Palladium, Zinn, Blei, Titan, Rubidium, Vanadium, Gallium, Terbium, Cer, Lanthan und Zink ausgewählt wird.

13. Vorrichtung nach Anspruch 11, in der es sich bei dem Metall um Kupfer handelt.

14. Vorrichtung nach Anspruch 11, in der Harnstoff als Stickstoffquelle verwendet wird.

15. Vorrichtung nach Anspruch 11, die unter Harnstoff- oder Ammoniakatmosphäre verwendet wird.

**16.** Vorrichtung nach Anspruch 11, die in Gegenwart eines unter Ammoniummolybdat, DBU und DBN ausgewählten Katalysators verwendet wird.

**17.** Vorrichtung nach Anspruch 11, in der es sich bei dem Lösungsmittel um einen halogenierten aromatischen Kohlenwasserstoff, ausgewählt unter Alkylbenzolen, N-Methyl-2-pyrrolidon, Chinolinen, Trichlorbenzol und 1-Chlornaphthalin, oder einen Alkohol, ausgewählt unter Isoamylalkohol, n-Octanol, 2-Ethylhexanol und Ethylenglykol, handelt.

**18.** Vorrichtung nach Anspruch 11, in der die Mischung unter Verwendung von Mikrowellenenergie mit einer Rate von etwa 2~20˚C/Minute auf 120˚C erhitzt und ferner mit einer Rate von etwa 0,25~10˚C/Minute auf eine Endherstellungstemperatur von 130~250˚C erhitzt wird.

**Revendications**

**1.** Procédé pour préparer une phtalocyanine métallique ou non métallique en traitant de l'acide phtalique anhydre, du phtalimide, de la 1,3-diiminoisoindoline, du 1,2-dicyanobenzène, un dérivé halogéné de ceux-ci, un dérivé alkylé de ceux-ci ou un dérivé alcoxylé de ceux-ci avec des micro-ondes et des ondes ultrasonores, éventuellement en présence d'un chlorure métallique ou d'un alcoxymétal.

**2.** Procédé selon la revendication 1 dans lequel est employé un solvant.

**3.** Procédé selon la revendication 1 mis en oeuvre en l'absence de solvant.

**4.** Procédé selon la revendication 1 dans lequel le métal est choisi dans le groupe constitué par le cuivre, le fer, le nickel, le cobalt, le manganèse, l'aluminium, le palladium, l'étain, le plomb, le titane, le rubidium, le vanadium, le gallium, le terbium, le cérium, le lanthane et le zinc.

**5.** Procédé selon la revendication 1 ou 4 dans lequel le métal est le cuivre.

**6.** Procédé selon la revendication 1 ou 4 dans lequel de l'urée est employée comme source d'azote.

**7.** Procédé selon la revendication 1 ou 4 dans lequel la réaction est réalisée sous une atmosphère d'urée ou d'ammoniac.

**8.** Procédé selon la revendication 1 ou 4 dans lequel la réaction est réalisée en présence d'un catalyseur choisi parmi le molybdate d'ammonium, le DBU et le DBN.

**9.** Procédé selon la revendication 1 ou 2 dans lequel le solvant est un hydrocarbure aromatique halogéné choisi parmi les alkylbenzènes, la N-méthyl-2-pyrrolidone, les quinolines, le trichlorobenzène et le 1-chloronaphtalène, ou un alcool choisi parmi l'alcool isoamylique, le n-octanol, le 2-éthylhexanol et l'éthylène glycol.

**10.** Procédé selon la revendication 1 ou 4, comprenant un chauffage à une vitesse d'environ 2-20˚C/minute jusqu'à 120˚C en utilisant de l'énergie micro-onde, et un chauffage supplémentaire à une vitesse d'environ 0,25-10˚C/minute jusqu'à une température de préparation finale de 130-250˚C.

**11.** Appareil pour préparer une phtalocyanine métallique ou non métallique comprenant : un magnétron (1) fournissant une fréquence de 0,1-100 GHz et une puissance de 100-3000 W ; un répartiteur d'ondes (3) pour rendre la longueur d'onde de micro-ondes uniforme dans une enceinte à micro-ondes (2) ; un régulateur de température PID (8) pour mesurer et contrôler précisément la température de réactifs ; un thermocouple de type K protégé des micro-ondes (4) ; un condensateur (5) et un agitateur (6) qui sont respectivement installés dans trois orifices formés au sommet de l'enceinte à micro-ondes (2) ; une sonde à ultrasons (7) installée dans un orifice formé dans le fond de l'enceinte à micro-ondes (2) ; un récipient en Pyrex (9) pour recevoir des réactifs ; et un réservoir de solvant (10), dans lequel de l'acide phtalique anhydre, du phtalimide, de la 1,3-diiminoisoindoline, du 1,2-dicyanobenzène, un dérivé halogéné de ceux-ci, un dérivé alkylé de ceux-ci ou un dérivé alcoxylé de ceux-ci est mélangé de façon homogène avec un chlorure métallique ou un alcoxymétal dans un solvant dans le récipient en Pyrex (9) à 130-250˚C pendant 0,25-15 heures en utilisant des micro-ondes à une fréquence de 0,1-100 GHz et une puissance de 100-3000 W et des ondes ultrasonores à une fréquence de 1-1000 GHz et une puissance de 100-5000 W, tout en contrôlant précisément la température des réactifs à l'aide du thermocouple de type K (4) et du régulateur de température PID (8).

12. Appareil selon la revendication 11 dans lequel le métal est choisi dans le groupe constitué par le cuivre, le fer, le nickel, le cobalt, le manganèse, l'aluminium, le palladium, l'étain, le plomb, le titane, le rubidium, le vanadium, le gallium, le terbium, le cérium, le lanthane et le zinc.

13. Appareil selon la revendication 11 dans lequel le métal est le cuivre.

14. Appareil selon la revendication 11 dans lequel de l'urée est employée comme source d'azote.

15. Appareil selon la revendication 11 utilisé sous une atmosphère d'urée ou d'ammoniac.

16. Appareil selon la revendication 11 utilisé en présence d'un catalyseur choisi parmi le molybdate d'ammonium, le DBU et le DBN.

17. Appareil selon la revendication 11 dans lequel le solvant est un hydrocarbure aromatique halogéné choisi parmi les alkylbenzènes, la N-méthyl-2-pyrrolidone, les quinolines, le trichlorobenzène et le 1-chloronaphtalène, ou un alcool choisi parmi l'alcool isoamylique, le n-octanol, le 2-éthylhexanol et l'éthylène glycol.

18. Appareil selon la revendication 11 dans lequel le mélange est chauffé à une vitesse d'environ 2-20˚C/minute jusqu'à 120˚C en utilisant de l'énergie micro-onde, et est encore chauffé à une vitesse d'environ 0,25-10˚C/minute jusqu'à une température de préparation finale de 130-250˚C.

FIG. 1

FIG. 2

FIG. 3

FIG. 4

FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP HEI8291261 B
- US 6491796 B

- WO 9954410 A

**Non-patent literature cited in the description**

- *Fifth International Electronic Conference on Synthetic Organic Chemistry,* 01 September 2001, 4-5